(19)

**Europäisches Patentamt**
**European Patent Office**
**Office européen des brevets**

(11) **EP 4 048 035 A1**

(12) **EUROPEAN PATENT APPLICATION**

(43) Date of publication:
24.08.2022 Bulletin 2022/34

(21) Application number: 22156247.3

(22) Date of filing: 11.02.2022

(51) International Patent Classification (IPC):
H05G 1/08 *(2006.01)*      H05G 1/34 *(2006.01)*

(52) Cooperative Patent Classification (CPC):
H05G 1/34; H05G 1/085

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 19.02.2021 JP 2021025437

(71) Applicant: FUJIFILM Corporation
Tokyo 106-8620 (JP)

(72) Inventor: SATO, Masaru
Kanagawa (JP)

(74) Representative: Kudlek, Franz Thomas
Dehns Germany
Theresienstraße 6-8
80333 München (DE)

(54) **CONTROL DEVICE, RADIOGRAPHY SYSTEM, CONTROL METHOD, AND CONTROL PROGRAM**

(57) Provided are a control device, a radiography system, a control method, and a control program that can shorten a calibration interval of a gate voltage supplied to a gate electrode of a radiation tube as compared to the related art.

Within one imaging period, a processor of a control device supplies a gate voltage, performs control to correct a voltage value of the gate voltage on the basis of a difference between a current value of an estimated anode current, which flows from a power supply voltage generator to an anode unit and is estimated on the basis of a detection value of a cathode current flowing from a cath-ode unit to a ground and a detection value of a gate current flowing from the power supply voltage generator to a gate electrode, and a target current value of an anode current set for an n-th imaging operation, and performs control to set the voltage value of the gate voltage corresponding to a corrected voltage value of the gate voltage corrected at the end of the n-th imaging operation and a target current value of the anode current set for an (n+1)-th imaging operation as a voltage value of the gate voltage which is supplied to the gate electrode first in the (n+1)-th imaging operation.

EP 4 048 035 A1

**Description**

**BACKGROUND OF THE INVENTION**

1. Field of the Invention

The present disclosure relates to a control device, a radiography system, a control method, and a control program.

2. Description of the Related Art

A radiography system that captures a radiographic image of an object is known. The radiography system has a radiation tube that generates radiation. As the radiation tube, in addition to a radiation tube that heats a filament as an electrode to emit thermal electrons, a radiation tube comprising a cold cathode that emits electrons without heating the electrode is known. The radiation tube comprises an electron emitting unit constituting a cold cathode and an anode unit (see, for example, WO2019/151251A). The electron emitting unit includes a cathode unit and a gate electrode for applying a voltage to the cathode unit. The anode unit has an anode surface facing the cathode unit, and a power supply voltage is supplied from a power supply voltage generator to the anode unit.
In the radiation tube, even in a case in which a gate voltage supplied to the gate electrode is the same, a current value of an anode current that actually flows to the anode unit may change due to the influence of a change over time or the like. Therefore, WO2019/151251A discloses a technique which performs calibration for correcting a gate voltage supplied to a gate electrode in order to set a current value of an anode current actually flowing to an anode unit to a desired value.

**SUMMARY OF THE INVENTION**

The calibration is a process related to the suppression of an error between a set target value and a measured value for the dose (so-called mAs value) of radiation emitted from the radiation tube, and the error between the target value and the measured value affects the quality of a radiographic image. Therefore, it is necessary to perform the calibration at least several times a year such that the error between the target value and the measured value is not too large.
As a calibration interval becomes longer, the error between the target value and the measured value becomes larger. In the field that particularly require the accuracy of managing the dose, there is a demand for shortening the calibration interval as much as possible to suppress the error as much as possible. For example, in the medical field, the management of the dose is important and requires accuracy. In the related art, since calibration is performed in a state in which the operation of the radiography system is stopped, for example, at night, there is a problem that it is difficult to shorten the calibration interval.
The present disclosure has been made in view of the above-mentioned problems, and an object of the present disclosure is to provide a control device, a radiography system, a control method, and a control program that can shorten a calibration interval of a gate voltage supplied to a gate electrode of a radiation tube as compared to the related art.
In order to achieve the above object, according to a first aspect of the present disclosure, there is provided a control device that controls a radiation tube which includes an electron emitting unit having a gate electrode and a cathode unit, to which a ground potential is supplied, and an anode unit, which has an anode surface facing the cathode unit and to which a power supply voltage is supplied from a power supply voltage generator, and emits radiation to an object in a case in which a radiographic image of the object is captured. The control device comprises at least one processor. Within one imaging period for which the radiation tube continues to emit the radiation to capture one radiographic image, the processor supplies a gate voltage to the gate electrode, performs control to correct a voltage value of the gate voltage on the basis of a difference between a current value of an estimated anode current, which flows from the power supply voltage generator to the anode unit and is estimated on the basis of a detection value of a cathode current flowing from the cathode unit to a ground and a detection value of a gate current flowing from the power supply voltage generator to the gate electrode, and a target current value of an anode current set for an n-th imaging operation, and performs control to set the voltage value of the gate voltage corresponding to a corrected voltage value of the gate voltage corrected at the end of the n-th imaging operation and a target current value of the anode current set for an (n+1)-th imaging operation as a voltage value of the gate voltage which is supplied to the gate electrode first in the (n+1)-th imaging operation.
According to a second aspect of the present disclosure, in the control device according to the first aspect, in a case in which the target current value of the anode current in the (n+1)-th imaging operation is matched with the target current value of the anode current in the n-th imaging operation, the processor may perform control to set the voltage value of the gate voltage corrected at the end of the n-th imaging operation as the voltage value of the gate voltage supplied to the gate electrode first in the (n+1)-th imaging operation.

According to a third aspect of the present disclosure, in the control device according to the first aspect or the second aspect, in a case in which the difference exceeds a preset threshold value, the processor may perform notification.

According to a fourth aspect of the present disclosure, in the control device according to any one of the first to third aspects, the processor may be capable of referring to correspondence relationship information indicating a correspondence relationship between the voltage value of the gate voltage and the current value of the anode current, acquire the voltage value of the gate voltage corresponding to the target current value set for the n-th imaging operation as a target voltage value on the basis of the correspondence relationship information, start the n-th imaging operation using the gate voltage with the acquired target voltage value as an initial gate voltage, and update the correspondence relationship information to correspondence relationship information in which the corrected voltage value of the gate voltage corrected at the end of the n-th imaging operation and the target current value correspond to each other.

According to a fifth aspect of the present disclosure, in the control device according to the fourth aspect, the processor may update a voltage value corresponding to the target voltage value in the correspondence relationship information to the corrected voltage value of the gate voltage corrected at the end of the n-th imaging operation as the update of the correspondence relationship information.

According to a sixth aspect of the present disclosure, in the control device according to any one of the first to third aspects, the processor may be capable of referring to correspondence relationship information indicating a correspondence relationship between the voltage value of the gate voltage and the current value of the anode current, acquire the voltage value of the gate voltage corresponding to the target current value set for the n-th imaging operation as a target voltage value on the basis of the correspondence relationship information, and supply a corrected voltage value obtained by correcting the target voltage value according to an amount of correction used to correct the gate voltage at the end of an (n-1)-th imaging operation as an initial gate voltage in the n-th imaging operation to the gate electrode.

According to a seventh aspect of the present disclosure, in the control device according to any one of the first to sixth aspects, the processor may repeat the control to correct the voltage value of the gate voltage within the one imaging period.

According to an eighth aspect of the present disclosure, in the control device according to any one of the first to seventh aspects, in a case in which the difference between the current value of the estimated anode current and the target current value is out of a preset allowable range, the processor may derive the corrected voltage value of the gate voltage by repeating a first process of adding or subtracting a predetermined amount of adjustment to adjust the voltage value of the gate voltage, a second process of supplying a gate voltage with the adjusted voltage value adjusted in the first process to the gate electrode, and a third process of acquiring the detection value of the cathode current, acquiring the detection value of the gate current, and estimating the current value of the estimated anode current after the second process until the difference falls within the allowable range.

According to a ninth aspect of the present disclosure, in the control device according to the eighth aspect, in a case in which the difference between the current value of the estimated anode current and the target current value does not fall within the preset allowable range within the imaging period of the n-th imaging operation, the processor may record information indicating that the correction of the gate voltage has not been completed within the imaging period.

In addition, in order to achieve the above object, according to a tenth aspect of the present disclosure, there is provided a radiography system comprising: a radiation tube; a radiography apparatus that irradiates an object with radiation emitted from the radiation tube to capture a radiographic image of the object; and the control device according to the present disclosure.

Further, in order to achieve the above object, according to an eleventh aspect of the present disclosure, there is provided a control method that is executed by a computer and controls a radiation tube which includes an electron emitting unit having a gate electrode and a cathode unit, to which a ground potential is supplied, and an anode unit, which has an anode surface facing the cathode unit and to which a power supply voltage is supplied from a power supply voltage generator, and emits radiation to an object in a case in which a radiographic image of the object is captured. The control method comprising: within one imaging period for which the radiation tube continues to emit the radiation to capture one radiographic image, supplying a gate voltage to the gate electrode; performing control to correct a voltage value of the gate voltage on the basis of a difference between a current value of an estimated anode current, which flows from the power supply voltage generator to the anode unit and is estimated on the basis of a detection value of a cathode current flowing from the cathode unit to a ground and a detection value of a gate current flowing from the power supply voltage generator to the gate electrode, and a target current value of an anode current set for an n-th imaging operation; and performing control to set the voltage value of the gate voltage corresponding to a corrected voltage value of the gate voltage corrected at the end of the n-th imaging operation and a target current value of the anode current set for an (n+1)-th imaging operation as a voltage value of the gate voltage which is supplied to the gate electrode first in the (n+1)-th imaging operation.

Furthermore, in order to achieve the above object, according to a twelfth aspect of the present disclosure, there is provided a control program that causes a computer to perform a process of controlling a radiation tube which includes an electron emitting unit having a gate electrode and a cathode unit, to which a ground potential is supplied, and an anode unit, which has an anode surface facing the cathode unit and to which a power supply voltage is supplied from a power supply voltage generator, and emits radiation to an object in a case in which a radiographic image of the object is captured. The control program causes the computer to perform a process comprising: within one imaging period for which the radiation tube continues to emit the radiation to capture one radiographic image, supplying a gate voltage to the gate electrode; performing control to correct a voltage value of the gate voltage on the basis of a difference between a current value of an estimated anode current, which flows from the power supply voltage generator to the anode unit and is estimated on the basis of a detection value of a cathode current flowing from the cathode unit to a ground and a detection value of a gate current flowing from the power supply voltage generator to the gate electrode, and a target current value of an anode current set for an n-th imaging operation; and performing control to set the voltage value of the gate voltage corresponding to a corrected voltage value of the gate voltage corrected at the end of the n-th imaging operation and a target current value of the anode current set for an (n+1)-th imaging operation as a voltage value of the gate voltage which is supplied to the gate electrode first in the (n+1)-th imaging operation.

According to the present disclosure, the calibration interval of the gate voltage supplied to the gate electrode of the radiation tube can be shorter than that in the related art.

## BRIEF DESCRIPTION OF THE DRAWINGS

Fig. 1 is a diagram schematically illustrating an example of the overall configuration of a radiography system according to an embodiment.
Fig. 2 is a block diagram illustrating an example of the configuration of a mammography apparatus and a console according to the embodiment.
Fig. 3 is a block diagram illustrating an example of the configuration of a radiation source according to the embodiment.
Fig. 4 is a functional block diagram illustrating an example of a radiation source control unit of the mammography apparatus according to the embodiment.
Fig. 5 is a diagram illustrating an example of correspondence relationship information according to the embodiment.
Fig. 6A is a diagram illustrating the correction of a gate voltage supplied to a gate electrode by a correction unit.
Fig. 6B is a diagram illustrating the correction of the gate voltage supplied to the gate electrode by the correction unit.
Fig. 7 is a flowchart illustrating an example of the flow of a radiation source control process by a radiation source control unit of the mammography apparatus according to the embodiment.
Fig. 8 is a flowchart illustrating another example of the flow of the radiation source control process by the radiation source control unit of the mammography apparatus according to the embodiment.

## DESCRIPTION OF THE PREFERRED EMBODIMENTS

Hereinafter, an embodiment of the present disclosure will be described in detail with reference to the drawings. In addition, this embodiment does not limit the present disclosure.

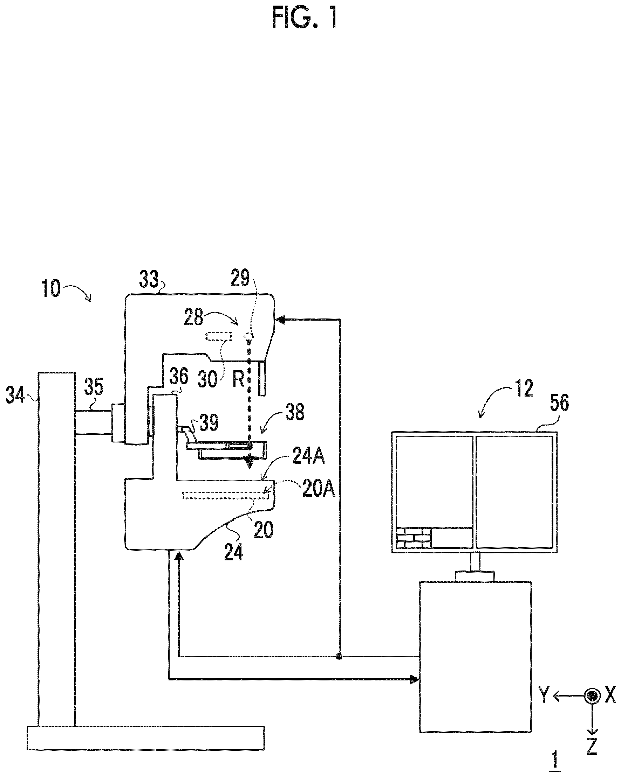

First, an example of the overall configuration of a radiography system according to this embodiment will be described. Fig. 1 is a diagram illustrating an example of the overall configuration of a radiography system 1 according to this embodiment. As illustrated in Fig. 1, the radiography system 1 according to this embodiment comprises a mammography apparatus 10 and a console 12.

First, the console 12 according to this embodiment will be described. The console 12 according to this embodiment has a function of controlling the mammography apparatus 10 using, for example, an imaging order and various kinds of information acquired from a radiology information system (RIS) through a wireless communication local area network (LAN) or the like and instructions input by a user, such as a doctor or a radiology technician, through an operation unit 56 or the like.

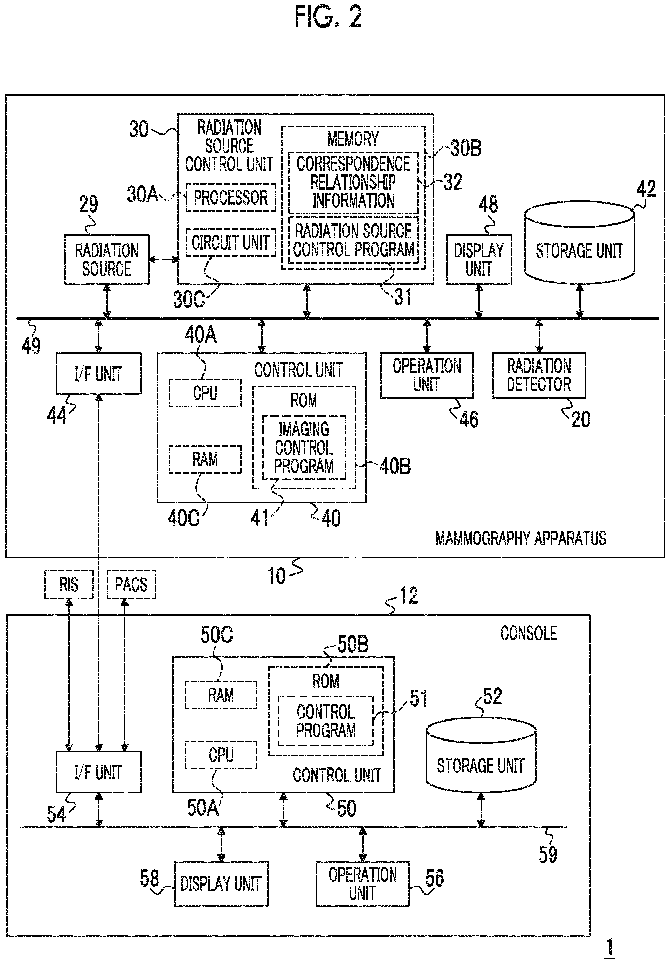

For example, the console 12 according to this embodiment is a server computer. Fig. 2 is a block diagram illustrating an example of the configuration of the mammography apparatus 10 and the console 12 according to the embodiment. As illustrated in Fig. 2, the console 12 comprises a control unit 50, a storage unit 52, an interface (I/F) unit 54, an operation unit 56, and a display unit 58. The control unit 50, the storage unit 52, the I/F unit 54, the operation unit 56, and the display unit 58 are connected to each other through a bus 59, such as a system bus or a control bus, such that they can transmit and receive various kinds of information.

The control unit 50 according to this embodiment controls the overall operation of the console 12. The control unit 50 comprises a central processing unit (CPU) 50A, a read only memory (ROM) 50B, and a random access memory (RAM) 50C. Various programs which are executed by the CPU 50A and include a control program 51 are stored in the ROM 50B in advance. The RAM 50C temporarily stores various kinds of data. The CPU 50A executes the control program 51 to control the capture of a radiographic image by the mammography apparatus 10.

For example, image data of the radiographic image captured by the mammography apparatus 10 and various other kinds of information are stored in the storage unit 52. A specific example of the storage unit 52 is a hard disk drive (HDD), a solid state drive (SSD), or the like.

The operation unit 56 is used by the user to input instructions, which are related to, for example, the capture of a radiographic image and include an instruction to emit the radiation R, various kinds of information, and the like. The operation unit 56 is not particularly limited. Examples of the operation unit 56 include various switches, a touch panel, a touch pen, and a mouse. The display unit 58 displays various kinds of information. In addition, the operation unit 56 and the display unit 58 may be integrated into a touch panel display.

The I/F unit 54 transmits and receives various kinds of information to and from the mammography apparatus 10, the RIS, and a picture archiving and communication system (PACS) using wireless communication or wired communication. In the radiography system 1 according to this embodiment, the console 12 receives the image data of the radiographic image captured by the mammography apparatus 10 from the mammography apparatus 10 through the I/F unit 54, using wireless communication or wired communication.

On the other hand, the mammography apparatus 10 according to this embodiment is an apparatus that is operated under the control of the console 12 and irradiates the breast of the subject as an object with radiation R (for example, X-rays) to capture a radiographic image of the breast. Fig. 1 is a side view illustrating an example of the outward appearance of the mammography apparatus 10 according to this embodiment. In addition, Fig. 1 illustrates an example of the outward appearance of the mammography apparatus 10 as viewed from the left side of the subject. In addition, the mammography apparatus 10 may be an apparatus that images the breast of the subject not only in a state in which the subject is standing (standing state) but also in a state in which the subject is sitting on, for example, a chair (including a wheelchair) (sitting state).

The radiation detector 20 detects the radiation R transmitted through the breast which is the object. Specifically, the radiation detector 20 detects the radiation R that has entered the breast of the subject and the imaging table 24 and reached the detection surface 20A of the radiation detector 20, generates a radiographic image on the basis of the detected radiation R, and outputs image data indicating the generated radiographic image. In the following description, in some cases, a series of operations of emitting the radiation R from the radiation source 29 and generating a radiographic image using the radiation detector 20 is referred to as "imaging". The type of the radiation detector 20 according to this embodiment is not particularly limited. For example, the radiation detector 20 may be an indirect-conversion-type radiation detector that converts the radiation R into light and converts the converted light into charge or a direct-conversion-type radiation detector that directly converts the radiation R into charge.

As illustrated in Fig. 1, the radiation detector 20 is disposed in the imaging table 24. In the mammography apparatus 10 according to this embodiment, in a case in which imaging is performed, the breast of the subject is positioned on an imaging surface 24A of the imaging table 24 by a user.

A compression plate 38 used to compress the breast in a case in which imaging is performed is attached to a compression unit 36 that is provided in the imaging table 24. Specifically, the compression unit 36 is provided with a compression plate driving unit (not illustrated) that moves the compression plate 38 in a direction (hereinafter, referred to as an "up-down direction") toward or away from the imaging table 24. A support portion 39 of the compression plate 38 is detachably attached to the compression plate driving unit and is moved in the up-down direction by the compression plate driving unit to compress the breast of the subject between the compression plate 38 and the imaging table 24.

The radiation emitting unit 28 comprises the radiation source 29 and a radiation source control unit 30. The radiation source 29 generates the radiation R under the control of the radiation source control unit 30 and emits the generated radiation R to the object (which will be described in detail below). The radiation source control unit 30 comprises a processor 30A, a memory 30B, and a circuit unit 30C. A radiation source control program 31 and correspondence relationship information 32 are stored in the memory 30B. In this embodiment, a CPU is used as an example of the processor 30A. The processor 30A executes the radiation source control program 31 stored in the memory 30B to control the radiation source 29. The radiation source control unit 30 according to this embodiment is an example of a control device according to the present disclosure. In addition, in this embodiment, the processor 30A is an example of a processor according to the present disclosure. Further, in this embodiment, the radiation source control program 31 is an example of a control program according to the present disclosure.

Furthermore, as illustrated in Fig. 1, the mammography apparatus 10 according to this embodiment comprises the imaging table 24, the arm portion 33, a base 34, and a shaft portion 35. The arm portion 33 is held by the base 34 so as to be movable in the up-down direction (Z-axis direction). Moreover, the arm portion 33 can be rotated with respect to the base 34 by the shaft portion 35. The shaft portion 35 is fixed to the base 34, and the shaft portion 35 and the arm portion 33 are rotated integrally.

Gears are provided in each of the shaft portion 35 and the compression unit 36 of the imaging table 24. The gears can be switched between an engaged state and a non-engaged state to switch between a state in which the compression unit 36 of the imaging table 24 and the shaft portion 35 are connected and rotated integrally and a state in which the shaft portion 35 is separated from the imaging table 24 and runs idle. In addition, components for switching between the transmission and non-transmission of the power of the shaft portion 35 are not limited to the gears, and various mechanical elements may be used.

Each of the arm portion 33 and the imaging table 24 can be relatively rotated with respect to the base 34, using the shaft portion 35 as a rotation axis. In this embodiment, engagement portions (not illustrated) are provided in each of the base 34, the arm portion 33, and the compression unit 36 of the imaging table 24. The state of the engagement portions is switched to connect each of the arm portion 33 and the compression unit 36 of the imaging table 24 to the base 34. One or both of the arm portion 33 and the imaging table 24 connected to the shaft portion 35 are integrally rotated on the shaft portion 35. The mammography apparatus 10 can perform simple imaging that captures an image of an object in a posture in which the radiation source 29 faces the radiation detector 20 and can also perform so-called tomosynthesis imaging that captures images a plurality of times while relatively moving the radiation source 29 with respect to the radiation detector 20 to change the irradiation angle of the radiation R with respect to the object. In a case in which the tomosynthesis imaging is performed in the mammography apparatus 10, the radiation source 29 of the radiation emitting unit 28 is moved to each of a plurality of irradiation positions having different irradiation angles by the rotation of the arm portion 33.

Further, as illustrated in Fig. 2, the mammography apparatus 10 according to this embodiment further comprises a control unit 40, a storage unit 42, an I/F unit 44, an operation unit 46, and a display unit 48 in addition to the radiation detector 20, the radiation source 29, and the radiation source control unit 30. The radiation detector 20, the radiation source 29, the radiation source control unit 30, the control unit 40, the storage unit 42, the I/F unit 44, the operation unit 46, and the display unit 48 are connected to each other through a bus 49, such as a system bus or a control bus, such that they can transmit and receive various kinds of information.

The control unit 40 controls the overall operation of the mammography apparatus 10 under the control of the console 12. The control unit 40 comprises a CPU 40A, a ROM 40B, and a RAM 40C. For example, the ROM 40B stores in advance various programs that are executed by the CPU 40A and include an imaging program for performing control related to the capture of radiographic images and an imaging control program 41 for performing control related to the capture of radiographic images. The RAM 40C temporarily stores various kinds of data.

For example, the image data of the radiographic image captured by the radiation detector 20 and various other kinds of information are stored in the storage unit 42. A specific example of the storage unit 42 is an HDD, an SSD, or the like. The I/F unit 44 transmits and receives various kinds of information to and from the console 12 using wireless communication or wired communication. The image data of the radiographic image captured by the radiation detector 20 in the mammography apparatus 10 is transmitted to the console 12 through the I/F unit 44 by wireless communication or wired communication.

Each of the control unit 40, the storage unit 42, and the I/F unit 44 according to this embodiment is provided in the imaging table 24.

In addition, the operation unit 46 is, for example, a plurality of switches that are provided in the imaging table 24 of the mammography apparatus 10 or the like. Further, the operation unit 46 may be provided as a touch panel switch or may be provided as a foot switch that is operated by the feet of the user such as a doctor or a radiology technician. The display unit 48 is provided in, for example, the imaging table 24 of the mammography apparatus 10 in order to display various kinds of information to the user.

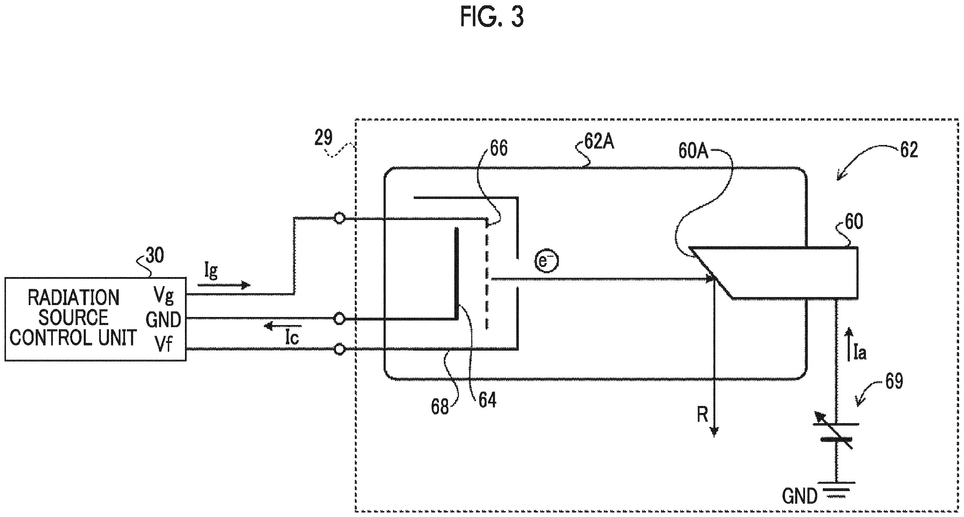

Next, the radiation source 29 and the radiation source control unit 30 of the mammography apparatus 10 according to this embodiment will be described in detail. Fig. 3 illustrates an example of the configuration of the radiation source 29. In addition, Fig. 3 illustrates the radiation source control unit 30.

As illustrated in Fig. 3, the radiation source 29 comprises a radiation tube 62 and a housing (not illustrated) that accommodates the radiation tube 62. The radiation tube 62 includes an envelope 62A, an anode unit 60, a cathode unit 64, a gate electrode 66, and a focus 68. The envelope 62A has, for example, a cylindrical shape and is made of, for example, glass or ceramic. The inside of the envelope 62A is hermetically sealed and kept in a vacuum state. A portion of the anode unit 60, the cathode unit 64, the gate electrode 66, and the focus 68 are accommodated in the envelope 62A.

The gate electrode 66 and the cathode unit 64 according to this embodiment are an example of an electrode emitting unit according to the present disclosure. For example, the cathode unit 64 is composed of a field emission array in which a plurality of electron emitting elements that emit electrons in a case in which an electric field is applied from the outside are arranged in a matrix. The electron emitting element has, for example, a conical shape with a sharp tip. For example, a spinto-type electron emitting element formed by the vapor deposition of molybdenum on a silicon substrate is used. The gate electrode 66 is an electrode for applying the electric field to the cathode unit 64. The gate electrode 66 has a plurality of opening portions which are formed so as to surround each electron emitting element and are arranged in a matrix so as to correspond to each electron emitting element. Electrons are emitted from the opening portions. The focus 68 is a focus electrode for focusing the electrons emitted by each electron emitting element of the cathode unit 64.

For example, a method for forming the gate electrode 66 and the cathode unit 64 is as follows. First, an oxide film which is a material forming the gate electrode 66 is formed on a silicon substrate, and a resist is formed on the oxide film according to a pattern of the gate electrode 66. After the resist is formed, the oxide film is etched to form an opening portion in the oxide film. A portion of the oxide film in which no resist is formed is the opening portion. After the opening portion is formed, a molybdenum film which is a material the electron emitting element is formed by vapor deposition. Therefore, a conical electron emitting element is formed in the opening portion. In Fig. 3, the cathode unit 64 and the gate electrode 66 are drawn so as to be separated from each other. However, in practice, the cathode unit 64 and the gate electrode 66 are formed on one substrate. For example, a carbon nanotube may be used as the material forming the electron emitting element.

A ground potential GND is supplied to the cathode unit 64 from the radiation source control unit 30, and a cathode current Ic flows from the cathode unit 64 to the radiation source control unit 30. In addition, a gate voltage Vg is supplied from the radiation source control unit 30 to the gate electrode 66, and the cathode current Ic flows from the cathode unit 64 to the radiation source control unit 30.

The anode unit 60 includes an anode surface 60A that is disposed so as to face the cathode unit 64. The anode unit 60 is provided with a target (not illustrated), and a power supply voltage higher than the ground potential GND is supplied from a power supply voltage generator 69 to the anode unit 60.

In a case in which the gate voltage is applied to the gate electrode 66 to turn on the gate electrode 66, an anode current Ia flows from the power supply voltage generator 69 to the anode unit 60 and then flows to the cathode unit 64. In this case, a plurality of electrons are emitted from the electron emitting element provided in the cathode unit 64. The emitted electrons are focused by the focus 66, collide with the anode surface 60A of the anode unit 60, pass through the inside of the anode unit 60, and are absorbed by the power supply voltage generator 69. The target provided on the anode surface 60A is made of a material that receives electrons and generates the radiation R. The radiation R is generated by the collision of the electrons emitted from the cathode unit 64 with the target provided on the anode surface 60A. As illustrated in Fig. 3, the generated radiation R is emitted in a direction corresponding to the inclination of the anode surface 60A. In addition, the anode current Ia is also called a tube current. The amount of radiation R generated by the radiation source 29 is adjusted by a mAs value which is the product of the tube current Ia and the irradiation time of the radiation R.

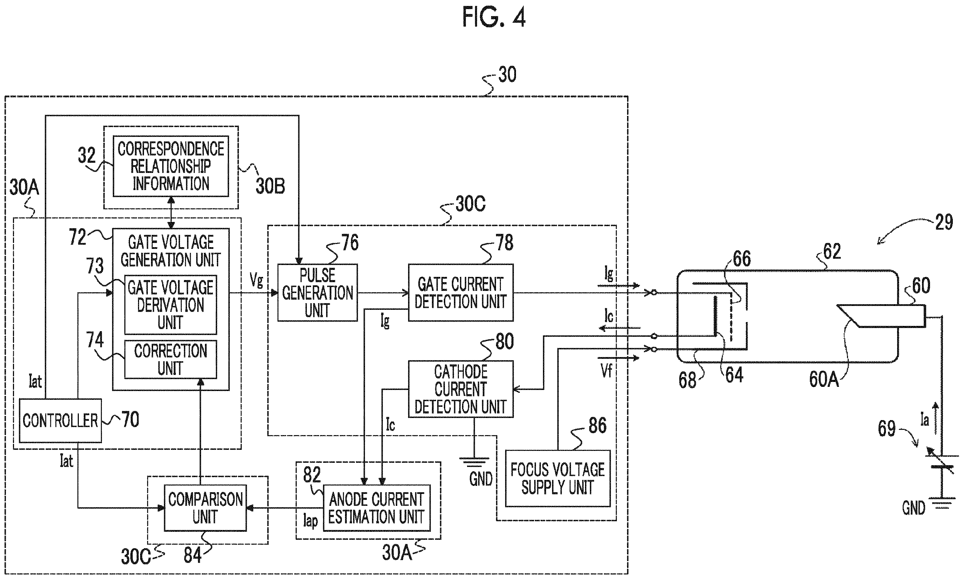

As described above, the focus 68 corrects the trajectory of the electrons emitted from the electron emitting element of the cathode unit 64 to focus the emitted electrons and is provided between the gate electrode 66 and the target of the anode surface 60A. The focus 68 is provided with a transmission portion, and the electrons transmitted through the transmission portion reach the target on the anode surface 60A. A focus voltage Vf is supplied to the focus 68 from a focus voltage supply unit 86 (see Fig. 4) of the radiation source control unit 30. The focal position of an electron beam on the anode surface 60A is adjusted according to the focus voltage Vf applied.

The radiation source control unit 30 has a function of supplying the ground potential GND to the cathode unit 64, a function of supplying the gate voltage Vg to the gate electrode 66, and a function of supplying the focus voltage Vf to the focus 68. In a case in which the radiation source control unit 30 starts to supply the gate voltage Vg to the gate electrode 66, the emission of the radiation R by the radiation source 29 is started. In a case in which the supply of the gate voltage Vg to the gate electrode 66 is stopped, the emission of the radiation R is stopped. Further, the radiation source control unit 30 has a function of controlling the current value of the anode current Ia flowing to the anode unit 60.

Fig. 4 illustrates an example of the functional blocks of the processor 30A and the circuit unit 30C of the radiation source control unit 30 according to this embodiment. As illustrated in Fig. 4, the processor 30A functions as a controller 70, a gate voltage generation unit 72, and an anode current estimation unit 82. As described above, the CPU executes the radiation source control program 31 stored in the memory 30B to implement the processor 30A according to this embodiment. Further, as illustrated in Fig. 4, the circuit unit 30C comprises a pulse generation unit 76, a gate current detection unit 78, a cathode current detection unit 80, a comparison unit 84, and the focus voltage supply unit 86. The pulse generation unit 76, the gate current detection unit 78, the cathode current detection unit 80, the comparison unit 84, and the focus voltage supply unit 86 are mainly implemented by analog circuits.

In addition, each of the units comprised in the radiation source control unit 30, such as the controller 70, the gate voltage generation unit 72, the pulse generation unit 76, the gate current detection unit 78, the cathode current detection unit 80, the anode current estimation unit 82, the comparison unit 84, and the focus voltage supply unit 86, may be implemented by a combination of hardware and an analog circuit, or either the hardware or the analog circuit. For example, not all but some of the controller 70, the gate voltage generation unit 72, and the anode current estimation unit 82 may be implemented by a combination of the CPU and the radiation source control program 31. Further, they may be implemented by various kinds of hardware, such as a field programmable gate array (FPGA), an application specific integrated circuit (ASIC), and an analog circuit, or the hardware and the CPU may be combined.

The controller 70 has a function of, for example, controlling each unit of the radiation source control unit 30. The controller 70 outputs a target current value (hereinafter, referred to as a "target value Iat") of the anode current Ia set for imaging to a gate voltage derivation unit 73 and the comparison unit 84. For example, the target value Iat is automatically set on the basis of the mAs value corresponding to imaging. Of course, the user may set the target value Iat on the basis of the mAs value. In addition, the controller 70 outputs a control signal for controlling the turn-on and turn-off of the output of the pulse generation unit 76 to the pulse generation unit 76.

The pulse generation unit 76 has a function of generating a gate voltage pulse having a height and a duty ratio corresponding to the voltage value (the supply value Vg will be described below) of the gate voltage Vg input from the gate voltage generation unit 72 and supplying the gate voltage pulse to the gate electrode 66 of the radiation source 29. In addition, in a case in which the gate voltage pulse is a single pulse, the "duty ratio" means a "pulse width". As described above, the control signal for controlling the turn-on and turn-off of the output is input from the controller 70 to the pulse generation unit 76. In a case in which the control signal for turning on the output is input to the pulse generation unit 76, the gate voltage pulse is output from the pulse generation unit 76, and the gate voltage Vg is supplied to the gate electrode 66. Therefore, the radiation R is emitted from the radiation source 29. Further, in a case in which the control signal for turning off the output is input to the pulse generation unit 76, the output of the gate voltage pulse by the pulse generation unit 76 is stopped, and the supply of the gate voltage Vg to the gate electrode 66 is stopped. Therefore, the emission of the radiation R by the radiation source 29 is stopped.

The gate current detection unit 78 is provided between the pulse generation unit 76 and the gate electrode 66 and has a function of detecting the current value of the gate current Ig flowing from the gate voltage derivation unit 73 to the gate electrode 66. Hereinafter, the current value of the gate current Ig detected by the gate current detection unit 78 is referred to as a "detection value Ig". The detection value Ig detected by the gate current detection unit 78 is output to the anode current estimation unit 82.

The cathode current detection unit 80 is provided between a supply unit that supplies the ground potential GND to the cathode unit 64 and the cathode unit 64 and has a function of detecting the current value of the cathode current Ic flowing from the cathode unit 64 to the supply unit that supplies the ground potential GND Hereinafter, the current value of the cathode current Ic detected by the cathode current detection unit 80 is referred to as a "detection value Ic". The detection value Ic detected by the cathode current detection unit 80 is output to the anode current estimation unit 82.

The anode current estimation unit 82 has a function of estimating the current value of the anode current Ia, which is estimated to flow from the power supply voltage generator 69 to the anode unit 60 on the basis of the detection value Ig input from the gate current detection unit 78 and the detection value Ic input from the cathode current detection unit 80, on the basis of the detection value Ig input from the gate current detection unit 78 and the detection value Ic input from the cathode current detection unit 80. As described above, the anode current estimation unit 82 does not detect the anode current Ia that actually flows from the power supply voltage generator 69 to the cathode unit 64, but estimates the current value of the anode current Ia, which is estimated to flow from the power supply voltage generator 69 to the cathode unit 64, from the detection value Ig and the detection value Ic. Therefore, in the following description, the anode current Ia is referred to as an "estimated anode current Iap", and the estimated current value is referred to as an "estimated value Iap".

Specifically, the anode current estimation unit 82 derives the estimated value Iap of the estimated anode current Iap using the following Expression (1):

$$\text{Estimated value Iap} = \text{Detection value Ic} - \text{Detection value Ig} \cdots (1).$$

The anode current estimation unit 82 outputs the estimated value Iap derived using the above-mentioned Expression (1) to the comparison unit 84.

The comparison unit 84 has a function of comparing the target value Iat input from the controller 70 with the estimated value Iap input from the anode current estimation unit 82 and outputting the comparison result to the gate voltage generation unit 72. For example, the comparison unit 84 according to this embodiment outputs information indicating a difference value Dv between the target value Iat and the estimated value Iap which is obtained using the following Expression (2) to the gate voltage generation unit 72:

$$\text{Difference value Dv} = \text{Target value Iat} - \text{Estimated value Iap} \cdots (2).$$

The gate voltage generation unit 72 has a function of supplying the gate voltage Vg to the gate electrode 66. The gate voltage generation unit 72 outputs the voltage value of the gate voltage Vg supplied to the gate electrode 66 to the pulse generation unit 76 to supply the gate voltage Vg to the gate electrode 66.

As illustrated in Fig. 4, the gate voltage generation unit 72 comprises the gate voltage derivation unit 73 and a correction unit 74. The gate voltage derivation unit 73 has a function of deriving the voltage value (hereinafter, referred to as a "supply value Vg") of the gate voltage Vg supplied to the gate electrode 66. In a case in which imaging is started, the target value Iat is input from the controller 70 as described above. The gate voltage derivation unit 73 can refer to correspondence relationship information 32 indicating the correspondence relationship between the voltage value of the gate voltage Vg and the current value of the anode current Ia and acquires the voltage value corresponding to the target value Iat to derive the supply value Vg which is the voltage value of the gate voltage Vg supplied to the gate electrode 66.

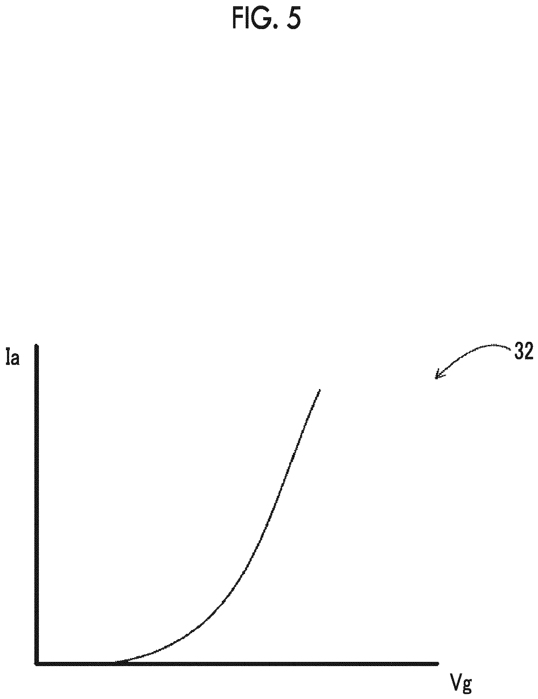

There is a correspondence relationship between the voltage value of the gate voltage Vg supplied to the gate electrode 66 and the current value of the anode current Ia flowing from the power supply voltage generator 69 to the anode unit 60. Fig. 5 illustrates an example of the correspondence relationship information 32 according to this embodiment. In this embodiment, a look-up table (LUT) illustrated in Fig. 5 is used as an example of the correspondence relationship information 32. In addition, even in a case in which the voltage value of the gate voltage Vg supplied to the gate electrode 66 is the same, the current value of the anode current Ia flowing from the power supply voltage generator 69 to the anode unit 60 may vary depending on the type or state of the radiation source 29. Therefore, the radiation source control unit 30 according to this embodiment stores a plurality of kinds of correspondence relationship information 32. Further, the correspondence relationship information 32 referred to by the gate voltage derivation unit 73 is defined according to, for example, a case in which the radiation source 29 is used for the first time or the correction result by the correction unit 74 which will be described below.

Even in a case in which the gate voltage Vg with the supply value Vg corresponding to the target value Iat input from the radiation source control unit 30, which is derived with reference to the correspondence relationship information 32, is supplied to the gate electrode 66, the current value of the anode current Ia that actually flows from the power supply voltage generator 69 to the anode unit 60 may be different from the target value Iat. For example, the correspondence relationships between the voltage value of the gate voltage Vg supplied to the gate electrode 66 and the current value of the anode current Ia flowing from the power supply voltage generator 69 to the anode unit 60 changes due to a change over time or the like according to the use of the radiation source 29. Therefore, the target value Iat and the estimated value Iap estimated by the anode current estimation unit 82 may be different from each other.

For this reason, the correction unit 74 has a function of correcting the supply value Vg derived by the gate voltage derivation unit 73 and supplying the corrected supply value Vg to the gate electrode 66 in order to match the target value Iat with the estimated value Iap.

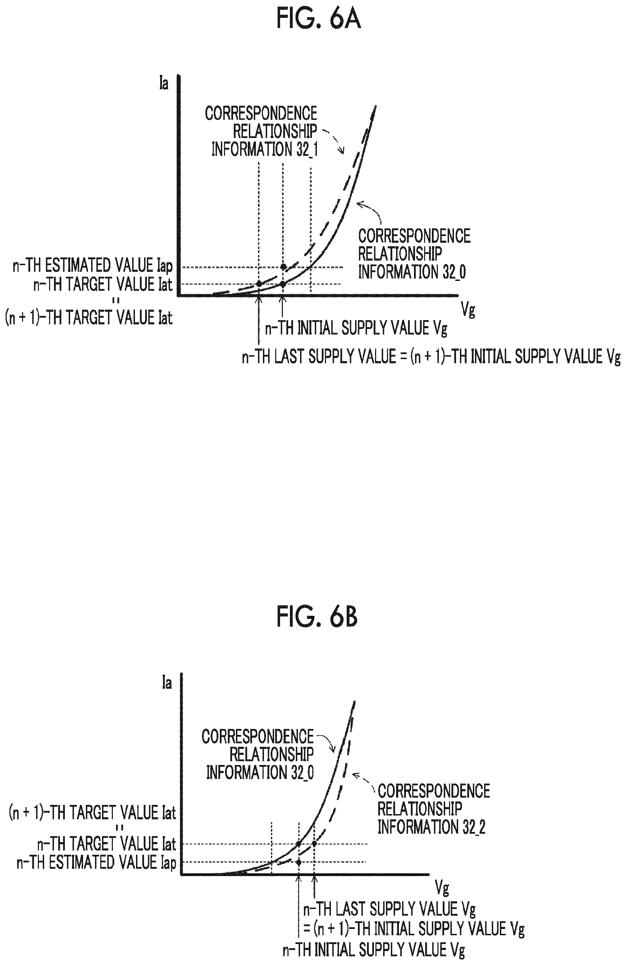

Fig. 6A illustrates a case in which an n-th estimated value Iap estimated by the anode current estimation unit 82 in a case in which the gate voltage derivation unit 73 supplies an n-th initial supply value Vg corresponding to the target value Iat to the gate electrode 66 first with reference to correspondence relationship information 32_0 in an n-th imaging operation is larger than an n-th target value Iat. With reference to the correspondence relationship information 32_0, the voltage value of the gate voltage Vg corresponding to the n-th estimated value Iap is larger than the n-th initial supply value Vg. Therefore, the supply value Vg supplied to the gate electrode 66 needs to be smaller than the n-th initial supply value Vg in order to set the current value of the anode current Ia, which actually flows from the power supply voltage generator 69 to the anode unit 60, as the target value Iat. For this reason, the correction unit 74 performs correction to increase the supply value Vg of the gate voltage Vg derived by the gate voltage derivation unit 73 in order to match the n-th target value Iat with the n-th estimated value Iap within the imaging period of the n-th imaging operation. Then, the corrected supply value Vg is output to the pulse generation unit 76 to supply the gate voltage Vg corresponding to the corrected supply value Vg to the gate electrode 66. The supply value Vg corrected by the correction unit 74 according to this embodiment corresponds to an example of a corrected voltage according to the present disclosure. In addition, in this embodiment, the "imaging period" means a period for which the radiation tube 62 continues to emit the radiation R in order to capture one radiographic image.

As described above, in a case in which the n-th target value Iat and the n-th estimated value Iap are different from each other, the estimated value Iap may be different from the target value Iat in an (n+1)-th imaging operation even though the supply value Vg corresponding to an (n+1)-th target value Iat is supplied as an (n+1)-th initial supply value Vg to the gate electrode 66 with reference to the correspondence relationship information 32_0.

Therefore, the correction unit 74 according to this embodiment corrects the n-th initial supply value Vg in the n-th imaging operation such that the supply value Vg in a case in which the target value Iat and the estimated value Iap are matched with each other is set as an n-th last supply value Vg, thereby updating the correspondence relationship information 32_0 to correspondence relationship information in which the n-th last supply value Vg and the n-th target value Iat correspond to each other. In the example illustrated in Fig. 6A, the correction unit 74 performs a process of updating the correspondence relationship information 32_0 to correspondence relationship information 32_1 in which the n-th last supply value Vg and the n-th target value Iat correspond to each other. As described above, since a plurality of kinds of correspondence relationship information 32 are stored in the radiation source control unit 30, for example, the correction unit 74 according to this embodiment performs control to select the correspondence relationship information 32_1 from the stored plurality of kinds of correspondence relationship information 32 and to set the correspondence relationship information 32_1 as the correspondence relationship information 32 used for the (n+1)-th imaging operation. Therefore, the correction unit 74 according to this embodiment performs control such that the supply value Vg of the gate voltage Vg corresponding to the supply value Vg of the gate voltage Vg corrected at the end of the n-th imaging operation and the target value Iat of the anode current set for the (n+1)-th imaging operation is set as the supply value Vg of the gate voltage Vg supplied to the gate electrode 66 first in the (n+1)-th imaging operation.

In addition, Fig. 6B illustrates a case in which the n-th estimated value Iap estimated by the anode current estimation unit 82 in a case in which the gate voltage derivation unit 73 supplies the n-th initial supply value Vg corresponding to the target value Iat to the gate electrode 66 first with reference to the correspondence relationship information 32_0 in the n-th imaging operation is smaller than the n-th target value Iat. With reference to the correspondence relationship information 32_0, the voltage value of the gate voltage Vg corresponding to the n-th estimated value Iap is smaller than the n-th initial supply value Vg. Therefore, the supply value Vg supplied to the gate electrode 66 needs to be larger than the n-th initial supply value Vg in order to set the current value of the anode current Ia, which actually flows from the power supply voltage generator 69 to the anode unit 60, as the target value Iat. Therefore, the correction unit 74 performs correction to increase the supply value Vg of the gate voltage Vg derived by the gate voltage derivation unit 73 in order to match the n-th target value Iat with the n-th estimated value Iap within the imaging period of the n-th imaging operation and outputs the corrected supply value Vg to the pulse generation unit 76 to supply the gate voltage Vg corresponding to the corrected supply value Vg to the gate electrode 66. The supply value Vg corrected by the correction unit 74 according to this embodiment corresponds to an example of a corrected voltage according to the present disclosure.

In this case, as described with reference to Fig. 6A, the correction unit 74 according to this embodiment corrects the n-th initial supply value Vg in the n-th imaging operation such that the supply value Vg in a case in which the target value Iat and the estimated value Iap are matched with each other is set as the n-th last supply value Vg, thereby updating the correspondence relationship information 32_0 to correspondence relationship information in which the n-th last supply value Vg and the n-th target value Iat correspond to each other. In the example illustrated in Fig. 6B, the correction unit 74 performs a process of updating the correspondence relationship information 32_0 to correspondence relationship information 32_2 in which the n-th last supply value Vg and the n-th target value Iat correspond to each other. As described above, since a plurality of kinds of correspondence relationship information 32 are stored in the radiation source control unit 30, for example, the correction unit 74 according to this embodiment performs control to select the correspondence relationship information 32_2 from the stored plurality of kinds of correspondence relationship information 32 and to set the correspondence relationship information 32_2 as the correspondence relationship information 32 used for the (n+1)-th imaging operation. Therefore, the correction unit 74 according to this embodiment performs control such that the supply value Vg of the gate voltage Vg corresponding to the supply value Vg of the gate voltage Vg corrected at the end of the n-th imaging operation and the target value Iat of the anode current set for the (n+1)-th imaging operation is set as the supply value Vg of the gate voltage Vg supplied to the gate electrode 66 first in the (n+1)-th imaging operation.

In the radiation source control unit 30 according to this embodiment, in either the case illustrated in Fig. 6A or the case illustrated in Fig. 6B, the correction unit 74 updates the correspondence relationship information 32 to the correspondence relationship information 32 in which the last supply value Vg and the n-th target value Iat in the n-th imaging operation correspond to each other to calibrate the radiation source 29 during the n-th imaging operation.

Next, the operation of controlling the radiation source 29 by the radiation source control unit 30 of the mammography apparatus 10 according to this embodiment will be described with reference to the drawings.

In a case in which a radiographic image of the breast of the subject is captured, the user positions the breast of the subject on the imaging table 24 of the mammography apparatus 10 and compresses the breast with the compression plate 38. After that, the user inputs an instruction to emit the radiation R using, for example, the operation unit 56 of the console 12. In a case in which the irradiation instruction is input, the console 12 outputs an imaging start signal to instruct the mammography apparatus 10 to start the capture of radiographic images.

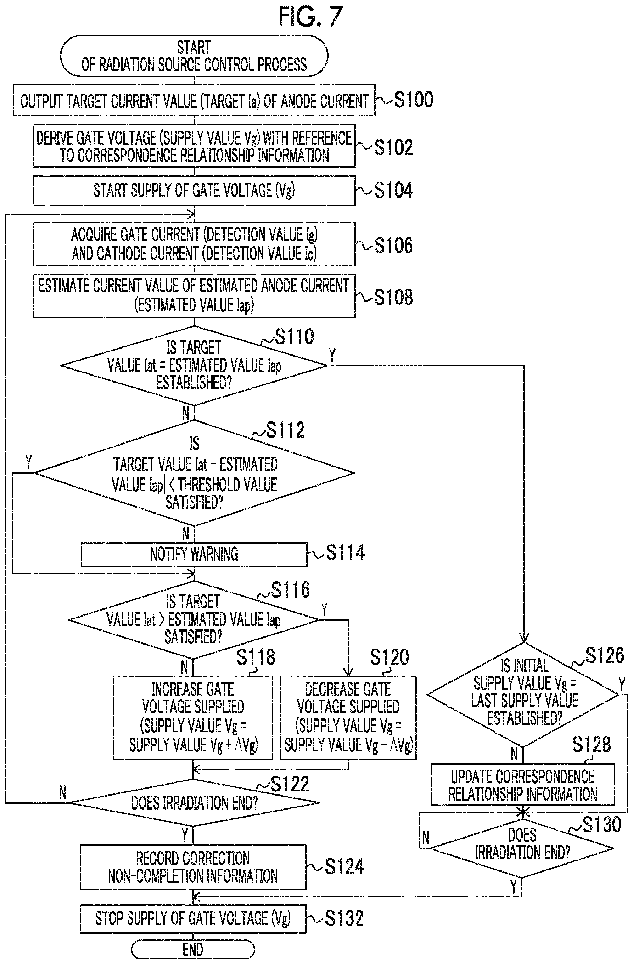

For example, the radiation source control unit 30 of the mammography apparatus 10 according to this embodiment executes the radiation source control program 31 to perform the radiation source control process. Fig. 7 is a flowchart illustrating an example of the flow of the radiation source control process by the radiation source control unit 30 of the mammography apparatus 10 according to this embodiment.

In Step S100 of the radiation control process illustrated in Fig. 7, the controller 70 outputs the target value Iat of the anode current Ia to the gate voltage generation unit 72. As described above, the controller 70 according to this embodiment acquires the target value Iat of the anode current Ia set for the current imaging operation and outputs the target value Iat to the gate voltage generation unit 72.

Then, in Step S102, the gate voltage derivation unit 73 of the gate voltage generation unit 72 derives the supply value Vg of the gate voltage Vg corresponding to the target value Iat input from the controller 70 with reference to the correspondence relationship information 32 as described above. The supply value Vg of the gate voltage Vg derived in this step is an example of a target voltage value according to the present disclosure.

Then, in Step S104, the gate voltage derivation unit 73 supplies the gate voltage Vg corresponding to the derived supply value Vg to the gate electrode 66. In this embodiment, as described above, the gate voltage generation unit 72 outputs the supply value Vg to the pulse generation unit 76. Further, the controller 70 outputs the control signal for turning on the output to the pulse generation unit 76. In a case in which the control signal for turning on the output is input from the controller 70, the pulse generation unit 76 generates a gate voltage pulse corresponding to the input supply value Vg and outputs the gate voltage pulse to the gate electrode 66.

In a case in which the gate voltage Vg is supplied to the gate electrode 66 in this way, as described above, electrons are emitted from the cathode unit 64 and collide with the target on the anode surface 60A of the anode unit 60, and the radiation R is generated in the radiation source 29. The gate current detection unit 78 detects the current value (detection value Ig) of the gate current Ig flowing from the gate voltage derivation unit 73 to the gate electrode 66 and outputs the current value to the anode current estimation unit 82. In addition, the cathode current detection unit 80 detects the current value (detection value Ic) of the cathode current Ic flowing from the cathode unit 64 to the supply unit that supplies the ground potential GND and outputs the current value to the anode current estimation unit 82.

Then, in Step S106, the anode current estimation unit 82 acquires the detection value Ig of the gate current Ig from the gate current detection unit 78 and acquires the detection value Ic of the cathode current Ic from the cathode current detection unit 80.

Then in Step S108, the anode current estimation unit 82 estimates the current value (estimated value Iap) of the estimated anode current. As described above, the anode current estimation unit 82 according to this embodiment derives the estimated value Iap of the estimated anode current Iap using the above-mentioned Expression (1). The anode current estimation unit 82 outputs the derived estimated value Iap to the comparison unit 84.

As described above, the comparison unit 84 compares the target value Iat input from the controller 70 with the estimated value Iap input from the anode current estimation unit 82 and outputs the difference value Dv derived by the above-mentioned Expression (2) as the comparison result to the gate voltage generation unit 72.

Then, in Step S110, the correction unit 74 compares the target value Iat input from the controller 70 with the estimated value Iap input from the anode current estimation unit 82 and determines whether or not the target value Iat and the estimated value Iap are matched with each other (the target value Iat = the estimated value Iap). In this embodiment, "the matching between the target value Iat and the estimated value Iap" is not limited to a case in which the target value Iat and the estimated value Iap are completely matched with each other, that is, a case in which the difference value Dv is "0". In this embodiment, a case in which the difference value Dv between the target value Iat and the estimated value Iap is within an allowable range such as an error range is also included in the case in which "the target value Iat and the estimated value Iap are matched with each other". In a case in which the target value Iat and the estimated value Iap are not matched with each other, the determination result in Step S110 is "No", and the process proceeds to Step S112. Specifically, in a case in which the difference value Dv is not "0", the process proceeds to Step S112.

In Step S112, the correction unit 74 determines whether or not the absolute value of the difference value Dv is smaller than a threshold value (|the target value Iat - the estimated value Iap| < the threshold value). In a case in which the absolute value of the difference value Dv is equal to or larger than the threshold value, the determination result in Step S112 is "No", and the process proceeds to Step S114.

Then, in Step S114, the comparison unit 84 issues a warning. For example, in a case in which the life of the radiation source 29 comes to an end, the difference value Dv between the target value Iat and the estimated value Iap increases. Therefore, in this embodiment, in a case in which the absolute value of the difference value Dv is equal to or larger than the threshold value, information indicating that the life of the radiation source 29 comes to an end or the end of the life of the radiation source 29 is approaching is notified as a warning. In addition, a notification method is not particularly limited. For example, either visible display using a display, a light emitting diode (LED), or the like or audible display using voice output from a speaker or the like may be performed using the display unit 48 of the mammography apparatus 10, the display unit 58 of the console 12, an external device of the radiography system 1, and the like. Further, the content of the warning is not particularly limited. For example, the content of the warning may be information warning that the radiation source 29 needs be replaced in order to maintain the quality of the captured radiographic image. Furthermore, the content of the notification in this step is not limited to the "warning" and may be, for example, a message simply informing the user that the end of the life of the radiation source 29 is approaching. In a case in which the process in Step S114 ends, the process proceeds to Step S116.

On the other hand, in a case in which the absolute value of the difference value Dv is smaller than the threshold value in Step S112, the determination result is "Yes", and the process proceeds to Step S116.

In Step S116, the correction unit 74 determines whether or not the target value Iat is larger than the estimated value Iap (the target value Iat > the estimated value Iap). In a case in which the target value Iat is larger than the estimated value Iap, the determination result in Step S114 is "Yes", and the process proceeds to Step S120. This case corresponds to the aspect illustrated in Fig. 6A and corresponds to a case in which the supply value Vg is large.

In Step S120, the correction unit 74 reduces the supply value Vg of the gate voltage Vg supplied to the gate electrode 66 by a predetermined amount $\Delta$Vg (the supply value Vg = the supply value Vg in the current imaging operation - $\Delta$Vg) and then proceeds to Step S122. In addition, $\Delta$Vg which is the amount of correction for the correction unit 74 to correct the supply value Vg is predetermined. Further, $\Delta$Vg which is the amount of correction may be the same value regardless of the difference value Dv. Furthermore, $\Delta$Vg which is the amount of correction may be a value corresponding to the difference value Dv. For example, in a case in which the difference value Dv is relatively large, $\Delta$Vg which is the amount of correction may be a relatively large value. As the difference value Dv becomes smaller, $\Delta$Vg which is the amount of correction may become smaller.

On the other hand, in Step S116, in a case in which the target value Iat is equal to or smaller than the estimated value Iap, the determination result in Step S116 is "No", and the process proceeds to Step S 118. This case corresponds to the aspect illustrated in Fig. 6B and corresponds to a case in which the supply voltage Vg is small.

In Step S118, the correction unit 74 increases the supply value Vg of the gate voltage Vg supplied to the gate electrode 66 by the predetermined amount ΔVg (the supply value Vg = the supply value Vg in the current imaging operation + ΔVg) and then proceeds to Step S122.

In Step S122, the correction unit 74 determines whether or not to end the emission of the radiation R. For example, in a case in which a time predetermined as the irradiation time of the radiation R has elapsed since the controller 70 output the control signal for turning on the output to the pulse generation unit 76, the correction unit 74 determines to end the emission of the radiation R. The determination result in Step S 122 is "No" until the emission of the radiation R ends, and the process returns to Step S106. Then, the processes in Steps S106 to S120 are repeated. On the other hand, in a case in which the emission of the radiation R ends, the determination result in Step S122 is "Yes", and the process proceeds to Step S124. Further, in this case, the emission of the radiation R may end before the estimated value Iap is matched with the target value Iat.

Then, in Step S124, the correction unit 74 records correction non-completion information indicating that the correction of the supply value Vg has not been completed on, for example, the radiation source control unit 30. The correction non-completion information recorded in this step can be referred to, for example, by the request of the user. Further, for example, in a case in which the next imaging operation is started, the user may be notified of the correction non-completion information.

On the other hand, in a case in which the target value Iat and the estimated value Iap are matched with each other in Step S110, the determination result is "Yes", and the process proceeds to Step S126. Specifically, in a case in which the difference value Dv is "0", the process proceeds to Step S126.

In Step S126, the correction unit 74 determines whether or not the supply value Vg supplied to the gate electrode 66 first in the current imaging operation is matched with the supply value Vg supplied at the end of the current imaging operation (the initial supply value Vg = the last supply value Vg). In other words, it is determined whether or not the n-th initial supply value Vg is the n-th last supply value Vg. In a case in which the initial supply value Vg is matched with the last supply value Vg, the determination result in Step S126 is "Yes", and the process proceeds to Step S130.

On the other hand, in a case in which the initial supply value Vg is not matched with the last supply value Vg, the determination result in Step S126 is "No", and the process proceeds to Step S128.

In Step S128, the correction unit 74 updates the correspondence relationship information 32. As described above, the correction unit 74 according to this embodiment performs either the process of updating the correspondence relationship information 32_0 to the correspondence relationship information 32_1 or the process of updating the correspondence relationship information 32_0 to the correspondence relationship information 32_2. In addition, in a case in which the estimated value Iap of the estimated anode current estimated by supplying the initial supply value Vg is matched with the target value Iat, this process may be omitted, or a process of specifying that the current correspondence relationship information 32 is used without any change may be performed.

Then, in Step S130, the correction unit 74 determines whether or not to end the emission of the radiation R. For example, the correction unit 74 performs the determination as in Step S122. The determination result in Step S130 is "No" until the emission of the radiation R ends. On the other hand, in a case in which the emission of the radiation R ends, the determination result in Step S130 is "Yes", and the process proceeds to Step S132.

In Step S132, the gate voltage generation unit 72 stops the supply of the gate voltage Vg to the gate electrode 66. In this embodiment, as described above, the gate voltage generation unit 72 stops the output of the supply value Vg to the pulse generation unit 76. Further, the controller 70 outputs the control signal for turning off the output to the pulse generation unit 76. The pulse generation unit 76 ends the generation of the gate voltage pulse in a case in which the control signal for turning off the output is input from the controller 70. Therefore, the generation of the radiation R in the radiation source 29 is stopped. In a case in which the process in Step S132 ends, the radiation source control process illustrated in Fig. 7 ends. In addition, the supply value Vg of the gate voltage Vg supplied to the gate electrode 66 in a case in which the emission of the radiation R is stopped in Step S132 in this embodiment is an example of a corrected voltage value according to the present disclosure.

Further, the aspect has been described in which the correction unit 74 updates the entire correspondence relationship information 32 in a case in which the correspondence relationship information 32 is updated to the correspondence relationship information 32 in which the last supply value Vg in the n-th imaging operation and the n-th target value Iat correspond to each other. For example, in the correspondence relationship information 32, only the supply value Vg of the gate voltage Vg corresponding to the n-th target value Iat may be corrected. Furthermore, as described above, in the aspect in which the entire correspondence relationship information 32 is updated, for example, the gate voltage derivation unit 73 can derive a more appropriate supply value Vg of the gate voltage Vg regardless of the (n+1)-th target value Iat, for example, even in a case in which the n-th target value Iat and the (n+1)-th target value Iat are different.

As described above, the radiation source control unit 30 of the mammography apparatus 10 according to the above-described embodiment comprises at least one processor. There is provided a control device that controls a radiation tube which includes an electron emitting unit having a gate electrode and a cathode unit, to which a ground potential is supplied, and an anode unit, which has an anode surface facing the cathode unit and to which a power supply voltage is supplied from a power supply voltage generator, and emits radiation to an object in a case in which a radiographic image of the object is captured. The control device comprises at least one processor. Within one imaging period for which the radiation tube continues to emit the radiation to capture one radiographic image, the processor supplies a gate voltage to the gate electrode, performs control to correct a voltage value of the gate voltage on the basis of a difference between a current value of an estimated anode current, which flows from the power supply voltage generator to the anode unit and is estimated on the basis of a detection value of a cathode current flowing from the cathode unit to a ground and a detection value of a gate current flowing from the power supply voltage generator to the gate electrode, and a target current value of an anode current set for an n-th imaging operation, and performs control to set the voltage value of the gate voltage corresponding to a corrected voltage value of the gate voltage corrected at the end of the n-th imaging operation and a target value of the anode current set for an (n+1)-th imaging operation as a voltage value of the gate voltage which is supplied to the gate electrode first in the (n+1)-th imaging operation.

As described above, according to the radiation source control unit 30 of the mammography apparatus 10 of this embodiment, the calibration interval of the gate voltage Vg supplied to the gate electrode 66 of the radiation tube 62 can be shorter than that in the related art.

In addition, in the above-described embodiment, the aspect in which the LUT is used as the correspondence relationship information 32 has been described. However, the correspondence relationship information 32 is not limited to this aspect. For example, the correspondence relationship information 32 may be a relational expression or the like indicating the correspondence relationship between the gate voltage Vg and the anode current Ia.

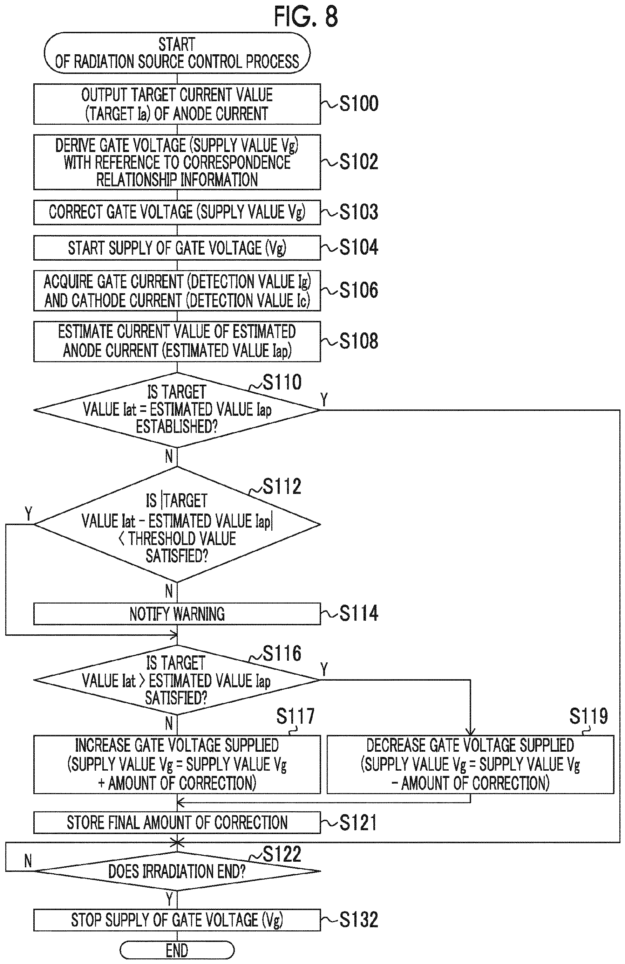

Further, in the above-described embodiment, the aspect in which the correction unit 74 adds or subtracts ΔVg to or from the supply value Vg of the gate voltage Vg to correct the supply value Vg has been described. However, the present disclosure is not limited to this aspect. For example, the correction may be performed once with a predetermined amount of correction. Fig. 8 is a flowchart illustrating an example of the radiation source control process in this case. The radiation source control process illustrated in Fig. 8 differs from the radiation source control process illustrated in Fig. 7 in that Step S103 is provided between Step S102 and Step S104.

In Step S103, the correction unit 74 performs a process of correcting the supply value Vg of the gate voltage Vg derived in Step S102. In the radiation source control process according to this embodiment, the supply value Vg of the gate voltage Vg which is the target voltage value derived in Step S102 is corrected by the amount of correction stored in Step S121 which will be described in detail below. In addition, in a case in which the initial value of the amount of correction is "0" and the radiation source control process is performed for the first time in the mammography apparatus 10, the correction unit 74 corrects the supply value Vg of the gate voltage Vg derived in Step S102 using the initial value.

Further, as illustrated in Fig. 8, Steps S117 and S119 are provided instead of Steps S118 and S120. Furthermore, the process in Step S121 is included before Step S122. In Step S117, the correction unit 74 increases the supply value Vg of the gate voltage Vg to be supplied by a predetermined amount of correction and then proceeds to Step S121. Moreover, in Step S119, the correction unit 74 decreases the supply value Vg of the gate voltage Vg to be supplied by a predetermined amount of correction and then proceeds to Step S121. The predetermined amount of correction used in Step S117 and Step S119 is the amount of correction predetermined by the experiment or the design. Examples of the amount of correction include the difference value Dv between the target value Iap and the estimated value Iap of the anode current Ia and the amount of correction obtained by the experiment or the design according to the target value Iat. In this case, instead of the correspondence relationship information 32, the difference value Dv between the target value Iap and the estimated value Iap of the anode current Ia and information indicating the correspondence relationship between the target value Iat and the amount of correction are stored in the memory 30B. Then, the correction unit 74 derives the difference value Dv between the target value Iap and the estimated value Iap of the anode current Ia in the current imaging operation and the amount of correction corresponding to the target value Iat with reference to the stored correspondence relationship and uses them for the correction in Step S117 or S119.

Further, in Step S121, the correction unit 74 stores the final amount of correction in the current imaging operation in the memory 30B or the like such that the amount of correction can be used for correction in the next imaging operation. In addition, the "final amount of correction" is a difference between the gate voltage Vg supplied to the gate electrode 66 at the beginning of the current imaging operation and the gate voltage Vg supplied to the gate electrode 66 in a case in which the correction ends. Specifically, the final amount of correction is an amount of correction obtained by adding the amount of correction used for the correction in Step S117 to the amount of correction used to correct the supply value Vg of the gate voltage Vg in Step S103 or an amount of correction obtained by subtracting the amount of correction used for the correction in Step S119 from the amount of correction used to correct the supply value Vg of the gate voltage Vg in Step S103. As described above, the storage of the final amount of correction makes it possible for the final amount of correction to be used in a case in which correction is performed in the next imaging operation.

As described above, in the radiation source control process illustrated in Fig. 8, the corrected voltage value obtained by correcting the target voltage value according to the amount of correction used to correct the gate voltage Vg at the end of an (n-1)-th imaging operation can be supplied to the gate electrode 66 as the initial gate voltage Vg in the n-th imaging operation. Therefore, even in this case, according to the radiation source control unit 30 of the mammography apparatus 10, the calibration interval of the gate voltage Vg supplied to the gate electrode 66 of the radiation tube 62 can be shorter than that in the related art.

In addition, in the above-described embodiment, the aspect in which the breast is applied as an example of the object according to the present disclosure and the mammography apparatus 10 is applied as an example of the radiography apparatus according to the present disclosure has been described. However, the object is not limited to the breast, and the radiography apparatus is not limited to the mammography apparatus. For example, the object may be the chest, the abdomen, or the like, and radiography apparatuses other than the mammography apparatus may be applied.

Further, in the above-described embodiment, the aspect in which the radiation source control unit 30 of the mammography apparatus 10 is an example of the control device according to the present disclosure has been described. However, devices other than the radiation source control unit 30 may have the functions of the control device according to the present disclosure. In other words, for example, another functional unit in the mammography apparatus 10, a device, such as the console 12, outside the mammography apparatus 10, or a device outside the radiography system 1 other than the radiation source control unit 30 may have some or all of the functions of the radiation source control unit 30.

In addition, in the above-described embodiment, for example, as described above, the following various processors can be used as the hardware structure of processing units performing various processes such as the gate voltage generation unit 72 and the anode current estimation unit 82. The various processors include, for example, a programmable logic device (PLD), such as a field programmable gate array (FPGA), that is a processor whose circuit configuration can be changed after manufacture and a dedicated electric circuit, such as an application specific integrated circuit (ASIC), that is a processor having a dedicated circuit configuration designed to perform a specific process, in addition to the CPU that is a generalpurpose processor which executes software (programs) to function as various processing units as described above.

One processing unit may be configured by one of the various processors or a combination of two or more processors of the same type or different types (for example, a combination of a plurality of FPGAs or a combination of a CPU and an FPGA). Further, a plurality of processing units may be configured by one processor.

A first example of the configuration in which a plurality of processing units are configured by one processor is an aspect in which one processor is configured by a combination of one or more CPUs and software and functions as a plurality of processing units. A representative example of this aspect is a client computer or a server computer. A second example of the configuration is an aspect in which a processor that implements the functions of the entire system including a plurality of processing units using one integrated circuit (IC) chip is used. A representative example of this aspect is a system-on-chip (SoC). As described above, various processing units are configured using one or more of the various processors as a hardware structure.

In addition, specifically, an electric circuit (circuitry) obtained by combining circuit elements, such as semiconductor elements, can be used as the hardware structure of the various processors.

In the above-described embodiment, the aspect in which the radiation source control program 31 is stored (installed) in the radiation source control unit 30 in advance has been described. However, the present disclosure is not limited thereto. The radiation source control program 31 may be recorded on a recording medium, such as a compact disc read only memory (CD-ROM), a digital versatile disc read only memory (DVD-ROM), or a universal serial bus (USB) memory, and then provided. In addition, the radiation source control program 31 may be downloaded from an external device through the network.

Explanation of References

1: radiography system

| 10: | mammography apparatus |
| 12: | console |
| 20: | radiation detector |
| 20A: | detection surface |
| 24: | imaging table |
| 24A: | imaging surface |
| 28: | radiation emitting unit |
| 29: | radiation source |
| 30: | radiation source control unit |

| | |
|---|---|
| 30A: | processor |
| 30B: | memory |
| 30C: | circuit unit |
| 31: | radiation source control program |
| 32, 32_0, 32_1: | correspondence relationship information |
| 33: | arm portion |
| 34: | base |
| 35: | shaft portion |
| 36: | compression unit |
| 38: | compression plate |
| 39: | support portion |
| 40A, 50A: | CPU |
| 40B, 50B: | ROM |
| 40C, 50C: | RAM |
| 41: | imaging control program |
| 42, 52: | storage unit |
| 44, 54: | I/F unit |
| 46, 56: | operation unit |
| 48: | display unit |
| 49, 59: | bus |
| 51: | control program |
| 58: | display unit |
| 60: | anode unit |
| 60A: | anode surface |
| 62: | radiation tube |
| 62A: | envelope |
| 64: | cathode unit |
| 66: | gate electrode |
| 68: | focus |
| 69: | power supply voltage generator |
| 70: | controller |
| 72: | gate voltage generation unit |
| 73: | gate voltage derivation unit |
| 74: | correction unit |
| 76: | pulse generation unit |
| 78: | gate current detection unit |
| 80: | cathode current detection unit |
| 86: | focus voltage supply unit |
| GND: | ground potential |
| Ia: | anode current |
| Iat: | target value |
| Iap: | estimated value |
| Ic: | cathode current |
| Ig: | gate current |
| R: | radiation |
| Vf: | focus voltage |
| Vg: | gate voltage |

**Claims**

1. A control device that controls a radiation tube which includes an electron emitting unit having a gate electrode and a cathode unit, to which a ground potential is supplied, and an anode unit, which has an anode surface facing the cathode unit and to which a power supply voltage is supplied from a power supply voltage generator, and emits radiation to an object in a case in which a radiographic image of the object is captured, the control device comprising:

   at least one processor,
   wherein, within one imaging period for which the radiation tube continues to emit the radiation to capture one radiographic image, the processor supplies a gate voltage to the gate electrode, performs control to correct a voltage value of the gate voltage on the basis of a difference between a current value of an estimated anode current, which flows from the power supply voltage generator to the anode unit and is estimated on the basis of a detection value of a cathode current flowing from the cathode unit to a ground and a detection value of a gate current flowing from the power supply voltage generator to the gate electrode, and a target current value of an anode current set for an n-th imaging operation, and performs control to set the voltage value of the gate voltage corresponding to a corrected voltage value of the gate voltage corrected at the end of the n-th imaging operation and a target current value of the anode current set for an (n+1)-th imaging operation as a voltage value of the gate voltage which is supplied to the gate electrode first in the (n+1)-th imaging operation.

2.  The control device according to claim 1,
    wherein, in a case in which the target current value of the anode current in the (n+1)-th imaging operation is matched with the target current value of the anode current in the n-th imaging operation, the processor performs control to set the voltage value of the gate voltage corrected at the end of the n-th imaging operation as the voltage value of the gate voltage supplied to the gate electrode first in the (n+1)-th imaging operation.

3.  The control device according to claim 1 or 2,
    wherein, in a case in which the difference exceeds a preset threshold value, the processor performs notification.

4.  The control device according to any one of claims 1 to 3,
    wherein the processor is capable of referring to correspondence relationship information indicating a correspondence relationship between the voltage value of the gate voltage and the current value of the anode current, acquires the voltage value of the gate voltage corresponding to the target current value set for the n-th imaging operation as a target voltage value on the basis of the correspondence relationship information, starts the n-th imaging operation using the gate voltage with the acquired target voltage value as an initial gate voltage, and updates the correspondence relationship information to correspondence relationship information in which the corrected voltage value of the gate voltage corrected at the end of the n-th imaging operation and the target current value correspond to each other.

5.  The control device according to claim 4,
    wherein the processor updates a voltage value corresponding to the target voltage value in the correspondence relationship information to the corrected voltage value of the gate voltage corrected at the end of the n-th imaging operation as the update of the correspondence relationship information.

6.  The control device according to any one of claims 1 to 3,
    wherein the processor is capable of referring to correspondence relationship information indicating a correspondence relationship between the voltage value of the gate voltage and the current value of the anode current, acquires the voltage value of the gate voltage corresponding to the target current value set for the n-th imaging operation as a target voltage value on the basis of the correspondence relationship information, and supplies a corrected voltage value obtained by correcting the target voltage value according to an amount of correction used to correct the gate voltage at the end of an (n-1)-th imaging operation as an initial gate voltage in the n-th imaging operation to the gate electrode.

7.  The control device according to any one of claims 1 to 6,
    wherein the processor repeats the control to correct the voltage value of the gate voltage within the one imaging period.

8.  The control device according to any one of claims 1 to 7,
    wherein, in a case in which the difference between the current value of the estimated anode current and the target current value is out of a preset allowable range, the processor derives the corrected voltage value of the gate voltage by repeating a first process of adding or subtracting a predetermined amount of adjustment to adjust the voltage value of the gate voltage, a second process of supplying a gate voltage with the adjusted voltage value adjusted in the first process to the gate electrode, and a third process of acquiring the detection value of the cathode current, acquiring the detection value of the gate current, and estimating the current value of the estimated anode current after the second process until the difference falls within the allowable range.

9.  The control device according to claim 8,
    wherein, in a case in which the difference between the current value of the estimated anode current and the target current value does not fall within the preset allowable range within the imaging period of the n-th imaging operation, the processor records information indicating that the correction of the gate voltage has not been completed within the imaging period.

10. A radiography system comprising:

a radiation tube;
a radiography apparatus that irradiates an object with radiation emitted from the radiation tube to capture a radiographic image of the object; and
the control device according to any one of claims 1 to 9.

11. A control method that is executed by a computer and controls a radiation tube which includes an electron emitting unit having a gate electrode and a cathode unit, to which a ground potential is supplied, and an anode unit, which has an anode surface facing the cathode unit and to which a power supply voltage is supplied from a power supply voltage generator, and emits radiation to an object in a case in which a radiographic image of the object is captured, the control method comprising:

within one imaging period for which the radiation tube continues to emit the radiation to capture one radiographic image,
supplying a gate voltage to the gate electrode;
performing control to correct a voltage value of the gate voltage on the basis of a difference between a current value of an estimated anode current, which flows from the power supply voltage generator to the anode unit and is estimated on the basis of a detection value of a cathode current flowing from the cathode unit to a ground and a detection value of a gate current flowing from the power supply voltage generator to the gate electrode, and a target current value of an anode current set for an n-th imaging operation; and
performing control to set the voltage value of the gate voltage corresponding to a corrected voltage value of the gate voltage corrected at the end of the n-th imaging operation and a target current value of the anode current set for an (n+1)-th imaging operation as a voltage value of the gate voltage which is supplied to the gate electrode first in the (n+1)-th imaging operation.

12. A computer-readable storage medium storing a control program that causes a computer to perform a process of controlling a radiation tube which includes an electron emitting unit having a gate electrode and a cathode unit, to which a ground potential is supplied, and an anode unit, which has an anode surface facing the cathode unit and to which a power supply voltage is supplied from a power supply voltage generator, and emits radiation to an object in a case in which a radiographic image of the object is captured, the control program causing the computer to perform a process comprising:

within one imaging period for which the radiation tube continues to emit the radiation to capture one radiographic image,
supplying a gate voltage to the gate electrode;
performing control to correct a voltage value of the gate voltage on the basis of a difference between a current value of an estimated anode current, which flows from the power supply voltage generator to the anode unit and is estimated on the basis of a detection value of a cathode current flowing from the cathode unit to a ground and a detection value of a gate current flowing from the power supply voltage generator to the gate electrode, and a target current value of an anode current set for an n-th imaging operation; and
performing control to set the voltage value of the gate voltage corresponding to a corrected voltage value of the gate voltage corrected at the end of the n-th imaging operation and a target current value of the anode current set for an (n+1)-th imaging operation as a voltage value of the gate voltage which is supplied to the gate electrode first in the (n+1)-th imaging operation.

# FIG. 1

# FIG. 2

MAMMOGRAPHY APPARATUS

30 RADIATION SOURCE CONTROL UNIT
30A PROCESSOR
30C CIRCUIT UNIT
MEMORY 30B
CORRESPONDENCE RELATIONSHIP INFORMATION 32
RADIATION SOURCE CONTROL PROGRAM 31
29 RADIATION SOURCE
48 DISPLAY UNIT
42 STORAGE UNIT
49
44 I/F UNIT
CONTROL UNIT 40A
CPU
RAM
40C
ROM
IMAGING CONTROL PROGRAM 40B
41 40
OPERATION UNIT 46
RADIATION DETECTOR 20
10

CONSOLE 12
RIS PACS
54 I/F UNIT
50C RAM
50B ROM
CONTROL PROGRAM 51
CPU
50A
CONTROL UNIT 50
52 STORAGE UNIT
59
58 DISPLAY UNIT
OPERATION UNIT 56

1

FIG. 3

EP 4 048 035 A1

FIG. 4

# FIG. 5

# FIG. 6A

# FIG. 6B

## FIG. 7

START
OF RADIATION SOURCE CONTROL PROCESS

OUTPUT TARGET CURRENT VALUE (TARGET Ia) OF ANODE CURRENT — S100

DERIVE GATE VOLTAGE (SUPPLY VALUE Vg) WITH REFERENCE
TO CORRESPONDENCE RELATIONSHIP INFORMATION — S102

START SUPPLY OF GATE VOLTAGE (Vg) — S104

ACQUIRE GATE CURRENT (DETECTION VALUE Ig)
AND CATHODE CURRENT (DETECTION VALUE Ic) — S106

ESTIMATE CURRENT VALUE OF ESTIMATED ANODE CURRENT
(ESTIMATED VALUE Iap) — S108

IS TARGET
VALUE Iat = ESTIMATED VALUE Iap
ESTABLISHED? — S110
Y

N

IS
|TARGET VALUE Iat − ESTIMATED
VALUE Iap| < THRESHOLD VALUE
SATISFIED? — S112
Y

N

NOTIFY WARNING — S114

IS TARGET
VALUE Iat > ESTIMATED VALUE Iap
SATISFIED? — S116
Y

N

INCREASE GATE
VOLTAGE SUPPLIED
(SUPPLY VALUE Vg =
SUPPLY VALUE Vg + ΔVg) — S118

DECREASE GATE
VOLTAGE SUPPLIED
(SUPPLY VALUE Vg =
SUPPLY VALUE Vg − ΔVg) — S120

IS INITIAL
SUPPLY VALUE Vg =
LAST SUPPLY VALUE
ESTABLISHED? — S126
Y

N

UPDATE CORRESPONDENCE
RELATIONSHIP INFORMATION — S128

DOES IRRADIATION END? — S122
N

Y

RECORD CORRECTION
NON-COMPLETION INFORMATION — S124

DOES
IRRADIATION END? — S130
N

Y

STOP SUPPLY OF GATE VOLTAGE (Vg) — S132

END

## FIG. 8

```
        ┌─────────────────────────────────────┐
        │              START                   │
        │ OF RADIATION SOURCE CONTROL PROCESS  │
        └─────────────────────────────────────┘
                         │
        ┌─────────────────────────────────────┐
        │      OUTPUT TARGET CURRENT VALUE     │ ～S100
        │      (TARGET Ia) OF ANODE CURRENT    │
        └─────────────────────────────────────┘
                         │
        ┌─────────────────────────────────────┐
        │  DERIVE GATE VOLTAGE (SUPPLY VALUE Vg)│
        │   WITH REFERENCE TO CORRESPONDENCE   │ ～S102
        │       RELATIONSHIP INFORMATION        │
        └─────────────────────────────────────┘
                         │
        ┌─────────────────────────────────────┐
        │  CORRECT GATE VOLTAGE (SUPPLY VALUE Vg)│ ～S103
        └─────────────────────────────────────┘
                         │
        ┌─────────────────────────────────────┐
        │    START SUPPLY OF GATE VOLTAGE (Vg) │ ～S104
        └─────────────────────────────────────┘
                         │
        ┌─────────────────────────────────────┐
        │ ACQUIRE GATE CURRENT (DETECTION VALUE Ig)│ ～S106
        │ AND CATHODE CURRENT (DETECTION VALUE Ic)│
        └─────────────────────────────────────┘
                         │
        ┌─────────────────────────────────────┐
        │   ESTIMATE CURRENT VALUE OF ESTIMATED│ ～S108
        │  ANODE CURRENT (ESTIMATED VALUE Iap) │
        └─────────────────────────────────────┘
                         │
                    ╱─────────╲  ～S110
                   ╱ IS TARGET ╲         Y
        ╲ VALUE Iat = ESTIMATED VALUE Iap ╱─────────→
                   ╲ ESTABLISHED? ╱
                    ╲───────────╱
                         │ N
                    ╱─────────╲  ～S112
         Y         ╱ IS |TARGET ╲
    ←──────── VALUE Iat − ESTIMATED VALUE Iap|
                  ╲ < THRESHOLD VALUE ╱
                   ╲  SATISFIED?  ╱
                    ╲───────────╱
                         │ N
        ┌─────────────────────────────────────┐
        │           NOTIFY WARNING            │ ～S114
        └─────────────────────────────────────┘
                         │
                    ╱─────────╲  ～S116
                   ╱ IS TARGET ╲        Y
        ╲ VALUE Iat > ESTIMATED VALUE Iap ╱─────────
                   ╲  SATISFIED?  ╱
                    ╲───────────╱
                         │ N        S117              S119
        ┌─────────────────────────┐  ┌─────────────────────────┐
        │ INCREASE GATE VOLTAGE    │  │ DECREASE GATE VOLTAGE    │
        │ SUPPLIED                 │  │ SUPPLIED                 │
        │ (SUPPLY VALUE Vg =       │  │ (SUPPLY VALUE Vg =       │
        │  SUPPLY VALUE Vg         │  │  SUPPLY VALUE Vg         │
        │  + AMOUNT OF CORRECTION) │  │  − AMOUNT OF CORRECTION) │
        └─────────────────────────┘  └─────────────────────────┘
                         │
        ┌─────────────────────────────────────┐
        │   STORE FINAL AMOUNT OF CORRECTION  │ ～S121
        └─────────────────────────────────────┘
                         │
         N          ╱─────────╲  ～S122
    ←──────────────╱ DOES IRRADIATION END? ╲
                    ╲───────────╱
                         │ Y
        ┌─────────────────────────────────────┐
        │  STOP SUPPLY OF GATE VOLTAGE (Vg)   │ ～S132
        └─────────────────────────────────────┘
                         │
                    ┌─────────┐
                    │   END   │
                    └─────────┘
```

Europäisches
Patentamt

European
Patent Office

Office européen
des brevets

# EUROPEAN SEARCH REPORT

Application Number

**EP 22 15 6247**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | WO 2019/151251 A1 (NANOX IMAGING PLC [GB]; NANOX JAPAN INC [JP]) 8 August 2019 (2019-08-08) * abstract; figures 1-3 * ----- | 1,2,4-6, 8,10-12 | INV. H05G1/08 H05G1/34 |
| Y | US 2007/237299 A1 (CARLSON TODD R [US] ET AL) 11 October 2007 (2007-10-11) * paragraphs [0034] - [0036] * ----- | 1,2,4-6, 8,10-12 | |
| A | US 2009/310845 A1 (OGAWA KOICHI [JP] ET AL) 17 December 2009 (2009-12-17) * paragraphs [0107] - [0109] * ----- | 1,2,4-6, 8,10-12 | |
| X | US 2017/118830 A1 (TOTSUKA YUKI [JP] ET AL) 27 April 2017 (2017-04-27) * figs. 1, 10-16 and related text * * abstract * ----- | 1-3,6-12 | |

TECHNICAL FIELDS
SEARCHED      (IPC)

H05G
H01J

The present search report has been drawn up for all claims

| Place of search | Date of completion of the search | Examiner |
|---|---|---|
| Munich | 1 July 2022 | Krauss, Jan |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
.................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 22 15 6247

This annex lists the patent family members relating to the patent documents cited in the above-mentioned European search report.
The members are as contained in the European Patent Office EDP file on
The European Patent Office is in no way liable for these particulars which are merely given for the purpose of information.

01-07-2022

| Patent document cited in search report | | Publication date | Patent family member(s) | | Publication date |
|---|---|---|---|---|---|
| WO 2019151251 | A1 | 08-08-2019 | CN | 111670611 A | 15-09-2020 |
| | | | JP | WO2019151251 A1 | 04-02-2021 |
| | | | WO | 2019151251 A1 | 08-08-2019 |
| US 2007237299 | A1 | 11-10-2007 | NONE | | |
| US 2009310845 | A1 | 17-12-2009 | EP | 1961383 A1 | 27-08-2008 |
| | | | US | 2009310845 A1 | 17-12-2009 |
| | | | WO | 2007046458 A1 | 26-04-2007 |
| US 2017118830 | A1 | 27-04-2017 | JP | 6852969 B2 | 31-03-2021 |
| | | | JP | 2017079858 A | 18-05-2017 |
| | | | US | 2017118830 A1 | 27-04-2017 |

EPO FORM P0459

For more details about this annex : see Official Journal of the European Patent Office, No. 12/82

**REFERENCES CITED IN THE DESCRIPTION**

**Patent documents cited in the description**

- WO 2019151251 A